# FASCICULE DE BREVET EUROPEEN

(11) **EP 3 075 308 B1**
(45) Date de publication et mention de la délivrance du brevet: **24.02.2021**
(21) Numéro de dépôt: 16163537.0
(22) Date de dépôt: 01.04.2016
(51) Int. Cl.: A61B 5/01, A61B 5/00, H01Q 1/22, H01Q 3/24, H04B 1/3827

(54) **DISPOSITIF D'ACQUISITION SANS FIL INOFFENSIF D'UNE MESURE PHYSIOLOGIQUE**
VORRICHTUNG ZUR UNBEDENKLICHEN DRAHTLOSEN ERFASSUNG VON PHYSIOLOGISCHEN MESSDATEN
DEVICE FOR HARMLESS WIRELESS ACQUISITION OF A PHYSIOLOGICAL MEASUREMENT

(30) Priorité: 02.04.2015 FR 1552848
(43) Date de publication de la demande: 05.10.2016
(73) Titulaire: E-Takescare, 78000 Versailles (FR)
(72) Inventeur: BOUGEROL, Antonin, 78150 Le Chesnay (FR)
(74) Mandataire: Plasseraud IP

(56) Documents cités:
- JP-A- 2010 197 245
- US-A- 4 747 413
- US-A1- 2005 024 275
- US-A1- 2011 213 559
- US-A1- 2012 029 308
- US-A1- 2015 035 680

## Description

La présente invention appartient au domaine des dispositifs communicants pour l'acquisition de mesures sur un corps vivant. Elle concerne en particulier un dispositif d'acquisition sans fil configuré pour que les ondes émises par ce dispositif soient inoffensives à l'égard du corps vivant sur lequel les mesures sont faites. L'invention est définie par les revendications 1-11.

Cette méthode est particulièrement avantageuse dans le cas d'un dispositif d'acquisition destiné à mesurer la température d'un nourrisson.

On entend par « corps vivant », toute entité vivante au sens biologique, c'est-à-dire capable de reproduction. Le corps d'un être humain, d'un animal ou encore d'une plante sont donc des exemples de corps vivant.

Dans la suite de la description, l'exemple d'une mesure faite sur le corps d'un être humain est détaillé.

L'acquisition de mesures par des dispositifs d'acquisition traditionnels sur un corps humain impose généralement un protocole spécifique, devant parfois être réalisé par un médecin ou un infirmier.

Ce protocole est généralement pénible pour le patient sur lequel la mesure est effectuée. En particulier, les mesures faites sur les nourrissons sont généralement accompagnées de pleurs et sont systématiquement sources d'une grande agitation du nourrisson.

En outre, la mise en œuvre du protocole impose généralement que les mesures soient faites à des instants différents. Il est en effet difficile de laisser les dispositifs d'acquisition traditionnels en place sur le patient afin d'avoir une mesure en continue.

Pour réduire ces désagréments, des dispositifs d'acquisition comportant au moins une antenne pour une connexion sans fil avec un terminal utilisateur ont pu être proposés. La connexion sans fil avec le terminal utilisateur simplifie le protocole de mesure. En outre, le dispositif d'acquisition peut être placé sur une partie du corps humain durant des périodes longues. En effet, l'absence de fils réduit sensiblement l'encombrement du dispositif d'acquisition et permet ainsi au patient d'effectuer d'autres activités pendant l'acquisition des mesures.

L'acquisition des données en continu et à distance rend possible un suivi précis et aisé de l'évolution d'une fièvre, d'une infection, ou encore des effets d'un médicament Le document US 2012/029308 représente un exemple de dispositif d'acquisition d'au moins une mesure physiologique d'un corps vivant comportant au moins une antenne pour une connexion sans fil avec un terminal utilisateur.

Toutefois, certaines études scientifiques relèvent le caractère néfaste des ondes émises par une antenne pour le corps humain. En particulier, les parents sont généralement peu disposés à exposer leur nourrisson à ces ondes.

En outre, le nombre de parties du corps humain au niveau desquelles les mesures peuvent être acquises de manière efficace est très restreint. Ces parties sont généralement proches d'organes importants tels que le cœur (mesure sous les aisselles), les organes génitaux (mesure au niveau du rectum) ou encore le cerveau (mesure dans la bouche). L'exposition de ces organes aux ondes émises par les ondes est ainsi particulièrement problématique.

La présente invention vient améliorer la situation.

A cet effet, un premier aspect de l'invention vise un dispositif d'acquisition d'au moins une mesure physiologique d'un corps vivant, comportant au moins deux antennes directives pour une connexion sans fil avec un terminal utilisateur (2), le dispositif étant destiné à être en contact avec le corps vivant suivant une surface de contact dans une position d'utilisation, dans lequel chacune des antennes du dispositif a une directivité définie par un vecteur de rayonnement (*̅v̅_̅{̅R̅}̅*̅) respectif comportant une direction de rayonnement et un sens de rayonnement, dans lequel, chacune des antennes est configurée pour rayonner selon la direction de rayonnement de son vecteur de rayonnement, le sens de rayonnement étant opposé à une portion du corps vivant sur laquelle elle est posée lorsque le dispositif est dans la position d'utilisation, dans lequel, la surface de contact est apte à être située sensiblement sous une aisselle du corps humain et à s'étendre sur une portion de peau du tronc du corps humain lorsque le dispositif est dans la position d'utilisation, dans lequel une première antenne directive est située au niveau d'une première extrémité du dispositif et une deuxième antenne directive est située au niveau d'une deuxième extrémité du dispositif, opposée à la première extrémité du dispositif, dans lequel les au moins deux antennes ont des directivités respectives distinctes, chaque antenne définissant un champ de rayonnement, et dans lequel le dispositif est apte à déterminer que le terminal utilisateur se situe dans le champ de rayonnement d'une antenne parmi les première et deuxième antennes, et dans lequel seule ladite antenne est activée.

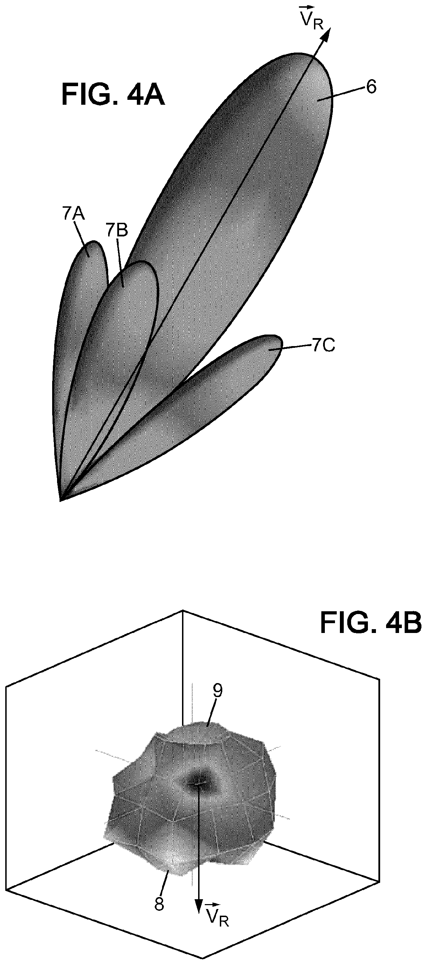

On entend par « directivité » de l'antenne, la capacité de l'antenne à exercer sa fonction d'émission d'ondes électromagnétiques suivant une ou plusieurs directions. Ici, la directivité est définie par un vecteur de rayonnement, ce qui signifie que les ondes émises par l'antenne rayonnent principalement à proximité du vecteur de rayonnement et dans le sens de ce vecteur. Le détail des caractéristiques techniques de l'antenne, et notamment de la directivité de l'antenne, est donné ci-après, notamment en référence aux figures 4A et 4B.

On entend par « sens de rayonnement (est) opposé à au moins une portion du corps vivant » que le vecteur s'écarte de la portion du corps vivant si bien qu'aucune intersection n'existe entre le vecteur et la portion.

Les ondes émises par l'antenne rayonnent dans un sens qui s'oppose à au moins une partie du corps vivant. Dès lors, le dispositif d'acquisition est totalement inoffensif pour ces parties du corps vivant. Il n'existe donc plus de contre-indications à l'utilisation d'un tel dispositif d'acquisition sans fil, quel que soit corps vivant sujet des mesures.

Dans un mode de réalisation, la mesure physiologique du corps vivant est une mesure de température.

On entend par « sensiblement sous une aisselle » tout voisinage situé sous l'aisselle. Ce voisinage peut comprendre une portion de peau située à proximité du creux de l'aisselle, une portion peau située à proximité d'un muscle pectoral, etc.

Bien que l'aisselle ne soit pas une partie du corps humain qui soit fermée et isolée de la température extérieure (comme le sont par exemple la bouche ou le rectum), il s'y trouve une artère sanguine principale (l'artère axiale). La présence de cette artère conduit à ce que la zone de l'aisselle soit une zone du corps qui soit très pertinente pour l'acquisition de diverses mesures physiologiques. Ainsi, la température au niveau de l'aisselle est très proche de la température effective du corps humain.

D'autre part, la zone située sensiblement sous l'aisselle et sur une portion de peau du tronc est particulièrement adaptée pour la pose du dispositif d'acquisition. Situé à cet endroit, le dispositif ne gêne pas les mouvements de la personne et est de plus très discret.

Chaque antenne définissant un champ de rayonnement, le dispositif est apte à déterminer que le terminal utilisateur se situe dans le champ de rayonnement d'une première antenne parmi les deux antennes, et seule la première antenne est activée. La nocivité électromagnétique du dispositif est encore réduite car seule une antenne sur les deux fonctionne. La consommation énergétique du dispositif est en outre améliorée.

Dans un mode de réalisation, une première antenne directive étant située au niveau d'une première extrémité du dispositif et une deuxième antenne directive étant située au niveau d'une deuxième extrémité du dispositif, opposée à la première extrémité du dispositif, avantageusement aucun obstacle tel que les bras n'est ainsi en mesure de perturber le fonctionnement des première et deuxième antennes. Les fréquences utilisées (e l'ordre du GHz) pour les transmissions sont en parties absorbées par le corps humain. Les antennes présentant une directivité spécifique, il convient en effet de ne pas interposer d'élément dans le chemin des ondes (du dispositif vers le terminal utilisateur).Dans un mode de réalisation, une première antenne est située au niveau d'une extrémité du dispositif apte à s'étendre sur une portion de peau située au-dessus d'un muscle pectoral lorsque le dispositif est dans la position d'utilisation et une deuxième antenne est située au niveau d'une autre extrémité du dispositif lorsque le dispositif est dans la position d'utilisation apte à s'étendre sur au moins un élément parmi :
- une portion de peau située au-dessus d'un muscle deltoïde ;
- une portion de peau située au-dessus d'un muscle térès majeur ;
- une portion de peau située au-dessus d'un muscle térès mineur ;
- une portion de peau située au-dessus d'un os omoplate.

Ainsi, lorsqu'un obstacle est présent entre une des antennes et le terminal, l'autre antenne peut être utilisée pour que la connexion avec le terminal soit maintenue.

En outre, les zones situées au-dessus d'un muscle deltoïde, au-dessus d'un muscle térès majeur, au-dessus d'un muscle térès mineur et/ou au-dessus d'un os omoplate sont également particulièrement adaptées pour augmenter le rendement de transmissions émises depuis une antenne situé au niveau de ces zones. En effet, ces zones sont planes (dos) et exemptes d'obstacles.

Dans un mode de réalisation, lorsque le dispositif est dans la position d'utilisation, la direction de rayonnement est définie par un axe sensiblement parallèle au plan médian du corps. L'antenne rayonne ainsi dans une direction et un sens opposés à toute partie du corps qui serait susceptible de subir les effets néfastes des ondes,

Dans un mode de réalisation, l'axe passe par l'aisselle. Ici aussi, la direction et le sens de rayonnement de l'antenne sont ainsi définis qu'ils réduisent au maximum les effets des ondes émises par l'antenne sur le corps.

Dans un mode de réalisation, le contact entre le dispositif et le corps vivant est apte à être assuré par une bande adhésive double face placée entre le dispositif et le corps vivant. Ce mode de fixation est discret, jetable, facile à mettre en œuvre et adapté à une utilisation sur un corps vivant.

Dans un mode de réalisation, le dispositif comprend un boîtier étanche comportant au moins un capteur de la mesure physiologique et ladite au moins une antenne. Ainsi, la transpiration, typiquement présente au niveau de l'aisselle, n'est pas susceptible de perturber le fonctionnement du dispositif. En outre, le dispositif est étanche aux produits chimiques de désinfection.

Dans un mode de réalisation, le boîtier est fait dans un matériau biocompatible. Ainsi, les risques de désagréments engendrés par le port du dispositif sont réduits. Un exemple de matériau biocompatible est le titane ou encore un matériau polymère de grade médical (en particulier un thermoplastique élastomère).

Dans un autre mode de réalisation, le dispositif comporte :
- au moins un premier capteur de température dont au moins une portion est apte à être en contact avec la peau du corps humain lorsque le dispositif est dans la position d'utilisation, pour mesurer une température cutanée ;
- au moins un deuxième capteur de température dont au moins une portion est apte à être située à l'extérieur du boîtier lorsque le dispositif est dans la position d'utilisation, pour mesurer une température extérieure au boîtier ;
- une unité de calcul pour pondérer la température cutanée en fonction de la température extérieure au boîtier.

Ainsi, la précision de la mesure de température cutanée est améliorée. L'influence de facteurs tels que la température ambiante, le port de vêtements et la position des bras sur la mesure de température cutanée est très importante. La mesure de température extérieure au boîtier donne une information pour pondérer les effets de ces facteurs.

Dans un mode de réalisation, le boîtier est réalisé dans une matière plastique flexible. Ainsi, le dispositif épouse les contours du corps vivant et améliore le confort du dispositif. Un matériau flexible pouvant être utilisé est un matériau de type thermoplastique élastomère (ou encore TPE).

Un deuxième aspect de l'invention concerne un procédé de transmission d'au moins une mesure physiologique d'un corps vivant (3), le corps vivant étant un corps humain, d'un dispositif d'acquisition vers un terminal utilisation, le dispositif comportant une antenne pour une connexion sans fil avec le terminal utilisateur, le dispositif étant destiné à être en contact avec le corps vivant suivant une surface de contact dans une position d'utilisation, dans lequel le dispositif comporte au moins deux antennes directives, dans lequel chacune des antennes a une directivité définie par un vecteur de rayonnement respectif comportant une direction de rayonnement et un sens de rayonnement, dans lequel une première antenne directive est située au niveau d'une première extrémité du dispositif et une deuxième antenne directive est située au niveau d'une deuxième extrémité du dispositif, opposée à la première extrémité du dispositif, dans lequel les au moins deux antennes ont des directivités respectives distinctes, chaque antenne définissant un champ de rayonnement, et dans lequel, lorsque le dispositif est dans la position d'utilisation, le procédé comporte les étapes de :
- acquisition (22) d'au moins une mesure physiologique du corps vivant ;
- détermination que le terminal utilisateur se situe dans le champ de rayonnement d'une antenne parmi les première et deuxième antennes ;
- génération (23) d'un signal radiofréquence comprenant ladite mesure ;
- émission (24) dudit signal par ladite antenne selon un sens de rayonnement opposé à au moins une portion du corps vivant, ladite antenne étant la seule antenne activée parmi les première et deuxième antennes.

Un troisième aspect de l'invention vise un programme informatique comportant des instructions pour la mise en œuvre du procédé selon le deuxième aspect de l'invention, lorsque ces instructions sont exécutées par un processeur d'un dispositif selon le premier aspect de l'invention.

D'autres caractéristiques et avantages de l'invention apparaîtront à l'examen de la description détaillée ci-après, et des dessins annexés sur lesquels:
- la figure 1 illustre un dispositif d'acquisition, selon un mode de réalisation de l'invention ;
- la figure 2 illustre une utilisation du dispositif, selon un mode de réalisation de l'invention ;
- la figure 3 représente des plans utilisés pour l'étude de l'anatomie du corps humain ;
- la figure 4A est un diagramme de rayonnement d'une antenne du dispositif, selon un mode de réalisation de l'invention ;
- la figure 4B est un diagramme de rayonnement d'une antenne du dispositif, selon un autre de mode de réalisation de l'invention ;
- la figure 5A est un schéma représentant des composants du dispositif, selon un mode de réalisation de l'invention ;
- la figure 5B illustre l'intérieur du dispositif, selon un mode de réalisation de l'invention ;
- la figure 6 est un diagramme illustrant les étapes d'un procédé de transmission, selon un mode de réalisation de l'invention.

L'invention est décrite ci-après dans une application, non limitative, à une mesure de température sur un corps humain. D'autres application, telles qu'une mesure d'un rythme cardiaque d'un sportif pendant un effort sont également envisageables.

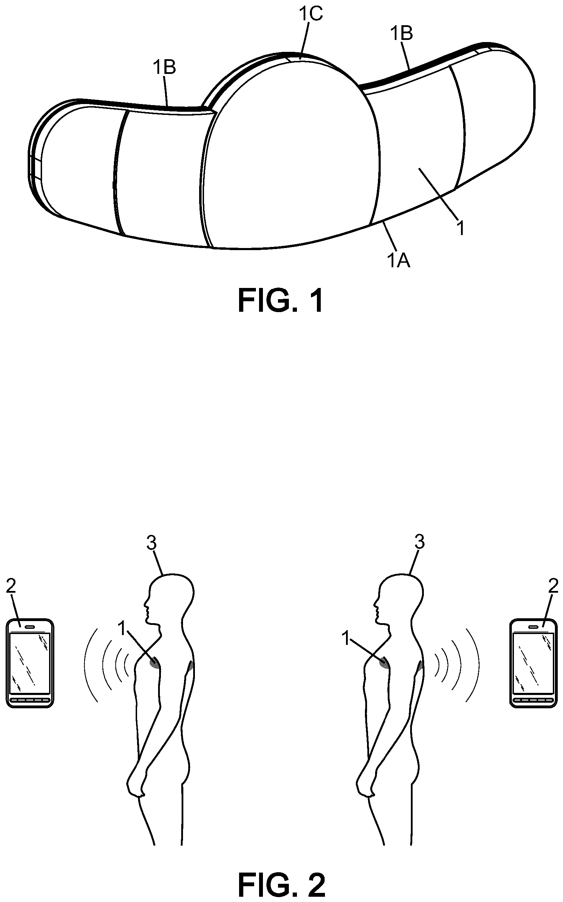

La **figure 1** représente l'extérieur d'un dispositif d'acquisition 1, selon un mode de réalisation.

Le dispositif comporte un boîtier en plastique présentant sensiblement une forme d'une portion d'un anneau (ou encore d'un boomerang à deux pales). On distingue un coté convexe 1B du boîtier correspondant à un rayon interne de la portion de l'anneau, d'un côté concave 1A du boîtier correspondant à un rayon externe de la portion de l'anneau. Un secteur angulaire correspondant à la portion d'anneau présente typiquement un angle de l'ordre de la dizaine de degrés. Le boîtier comporte en outre une excroissance 1C situé au niveau du coté convexe 1B du boîtier.

Le boîtier peut être flexible et/ou étanche. L'étanchéité peut être obtenue de plusieurs façons, en fonction du caractère démontable ou non du dispositif. Si la démontabilité n'est pas requise, le dispositif peut être enrobé dans une résine ou un élastomère flexible. En variante, le dispositif est constitué d'un boîtier en plusieurs parties (socle et couvercle), assemblées par soudage (technique par ultrason, laser ou lame chauffante) ou par collage. Si la démontabilité est requise, le boîtier peut alors être constitué de plusieurs parties à visser ou à clipser, et des joints doivent être utilisés.

La **figure 2** représente une personne humaine 3 utilisant le dispositif d'acquisition 1.

Lorsqu'il est utilisé pour acquérir et transmettre des mesures, le dispositif est en contact avec le corps humain suivant une surface de contact. La surface de contact est située sous une aisselle du corps humain et s'étend sur une portion de peau du tronc du corps humain.

Le dispositif 1 comporte en outre deux antennes pour qu'une connexion sans fil puisse être établie avec un terminal utilisateur 2. Le terminal utilisateur 2 est typiquement un smartphone, pour téléphone intelligent en français. Il peut également s'agir d'un dispositif dédié à la consultation des mesures physiologiques, d'un ordinateur personnel, etc.

Chacune des deux antennes a une directivité définie par un vecteur de rayonnement *̅v̅_̅{̅R̅}̅*̅ comportant une direction de rayonnement et un sens de rayonnement. Les aspects électromagnétiques relatifs à la directivité de l'antenne sont décrits ci-après en référence aux figures 4A et 4B.

En particulier, les antennes du dispositif sont déportées par rapport à une zone située immédiatement sous l'aisselle. Les zones d'émission du dispositif sont donc déportées des dessous de bras, ce qui réduit les interférences qui pourraient être causées par des obstacles tels que les bras ou le tronc du corps humain.

Ainsi, une première antenne est située au niveau d'une première extrémité du dispositif apte à s'étendre sur une portion de peau située au-dessus d'un muscle pectoral lorsque le dispositif est dans la position d'utilisation. La première extrémité peut également ne pas être en contact avec peau, tout en restant située dans un voisinage proche de la portion de peau située au-dessus muscle pectoral.

Une deuxième antenne est située au niveau d'une deuxième extrémité du dispositif lorsque le dispositif est dans la position d'utilisation. La deuxième extrémité est apte à s'étendre sur une portion de peau située au-dessus d'un muscle deltoïde, une portion de peau située au-dessus d'un muscle térès majeur, une portion de peau située au-dessus d'un muscle térès mineur et/ou une portion de peau située au-dessus d'un os omoplate. La deuxième antenne est située au niveau du dos de la personne portant le dispositif. Ici aussi la zone d'émission de la deuxième antenne est prévue pour réduire les obstacles et optimiser l'émission des ondes émises depuis la deuxième antenne. La deuxième extrémité peut également être située dans un voisinage proche de la portion de peau située au-dessus des muscles et os susmentionnés.

Pour un corps vivant autre qu'un corps humain, le dispositif est situé dans une zone comparable à celle, située sous une aisselle, décrite ci-dessus pour un être humain. Ainsi, le dispositif peut être situé au niveau de zones de raccords entre membres. Par exemple, le dispositif peut-être situé entre les pattes et le tronc d'un mammifère tel qu'un cochon. Dans le cas des plantes, le dispositif peut être situé au niveau d'une partie rigide (tronc, branche importante).

Le contact entre le dispositif et le corps vivant peut être assuré par une bande adhésive double face placée entre le dispositif et le corps vivant. Le mode de fixation est un élément déterminant pour le confort d'utilisation. Le dispositif utilise un adhésif double face médical pour maintenir le dispositif sur la peau. Cette solution permet de faciliter la mise en place du dispositif, d'être discrète et ne pas détériorer le confort d'utilisation.

Cet adhésif est constitué d'un film support en élastomère, ayant de part et d'autre des matières collantes différentes. La partie destinée à être collée sur le dispositif a un fort pouvoir d'adhérence, tandis que l'autre partie, collée sur la peau, est conçue pour limiter les irritations de peaux sensibles. Les deux parties collantes sont protégées par des films protecteurs, qui se retirent lors de l'utilisation. Cet adhésif permet le maintien du dispositif pendant plusieurs dizaines d'heures.

En variante, la fixation est assurée par des sangles faisant le tour du tronc du corps humain.

Pour que les ondes émises par l'antenne ne soient pas néfastes pour le corps humain, l'invention prévoit que le sens de rayonnement soit opposé à au moins une portion du corps, lorsque le dispositif est dans la position d'utilisation. En particulier, l'antenne est configurée pour que les ondes rayonnées par l'antenne soient émises dans un sens opposé aux organes vitaux du corps humain. L'antenne peut également être configurée pour que le sens de rayonnement soit opposé à d'autres organes tels que les organes génitaux, les seins, les ganglions lymphatiques, etc.

De plus, les deux antennes ont chacune des directivités spécifiques et définissent ainsi respectivement un premier et un deuxième champ de rayonnement.

La première antenne émet dans un sens opposé à une zone du corps humain sur laquelle elle est posée (la portion de peau située au-dessus du muscle pectoral) et la deuxième antenne dans un sens opposé à la zone du dos sur laquelle elle est posée. Ainsi, la première antenne émet vers l'avant de la personne humaine et la deuxième antenne émet vers l'arrière.

Lorsque le terminal utilisateur est situé dans l'un des champs de rayonnement, seule l'antenne à partir de laquelle ce champ de rayonnement est généré est activée. Pour ce faire, chacune des antennes est apte à émettre un signal de test. Si un terminal utilisateur reçoit l'un au moins des signaux de test, il renvoie un message de confirmation indiquant une mesure de puissance de réception du signal de test qui a été reçu. Le dispositif est alors apte à déterminer quelle antenne doit être utilisée pour que la connexion avec le terminal utilisateur soit assurée.

Sur la figure 2, deux situations sont représentées. A gauche, le terminal utilisateur est dans le champ de rayonnement de la première antenne. A droite, le terminal utilisateur est dans le champ de rayonnement de la deuxième antenne.

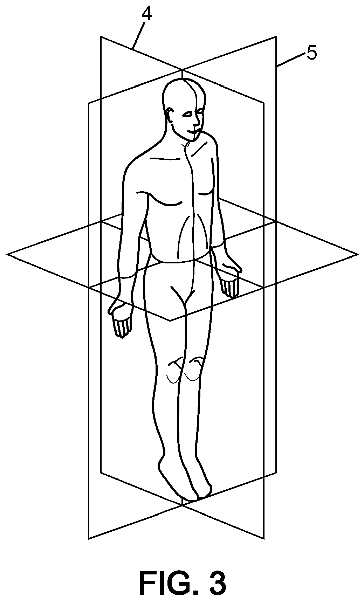

Le vecteur de rayonnement *̅v̅_̅{̅R̅}̅*̅ des antennes est choisi pour que les ondes générées par les antennes ne soient pas émises vers le corps humain. Ainsi, lorsque le dispositif est dans la position d'utilisation, la direction de rayonnement est définie par un axe sensiblement parallèle au plan médian du corps, représenté à la référence 4 de la **figure 3****.** En particulier, l'axe définissant la direction de rayonnement passe par l'aisselle et est perpendiculaire au plan transversal 5 du corps.

Des exemples de diagrammes de rayonnement des antennes du dispositif 1 sont représentés aux **figures 4A** et **4B****.**

Le champ de rayonnement représenté à la figure 4A comprend un lobe principal 6 et quatre lobes secondaires, dont trois sont représentés et référencés 7A, 7B et 7C. Le champ de rayonnement représenté à la figure 4A donne un exemple d'une antenne fortement directive, ce qui signifie que la puissance rayonnée par l'antenne est localisée dans un voisinage réduit du vecteur de rayonnement *̅v̅_̅{̅R̅}̅*̅.̅ On entend par « voisinage réduit », une zone proche du vecteur de rayonnement *̅v̅_̅{̅R̅}̅*̅, par exemple une zone comprise dans un cylindre ayant *̅v̅_̅{̅R̅}̅*̅ comme axe de révolution et un rayon de 0,5 à 10 cm (pour une antenne de faible puissance, typiquement destinée à émettre les mesures acquises par le dispositif).

La figure 4B donne au contraire un exemple d'une antenne moins directive. La puissance rayonnée par l'antenne de la figure 4B est en effet localisée dans un voisinage de taille supérieure à celle du voisinage de l'antenne de la figure 4A. Ce voisinage est par exemple compris dans un cylindre ayant *̅v̅_̅{̅R̅}̅*̅ comme axe de révolution et un rayon de plus de 10 cm (toujours pour une antenne de faible puissance).

Bien que le champ de rayonnement représenté à la figure 4B ne soit pas cantonné à une zone précise autour du vecteur de rayonnement *̅v̅_̅{̅R̅}̅*̅, il est toutefois limité à un sens de rayonnement précis. Sur la figure 4B, cela signifie que les ondes ne sont rayonnées par l'antenne que vers le bas, au niveau de la zone 8. Les ondes ne sont toutefois pas rayonnées vers le haut, au niveau de la zone 9.

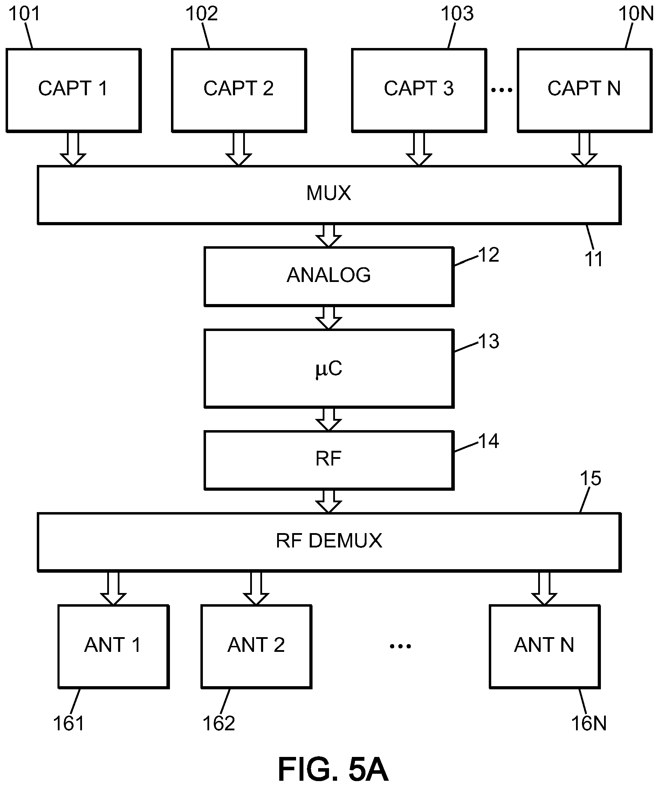

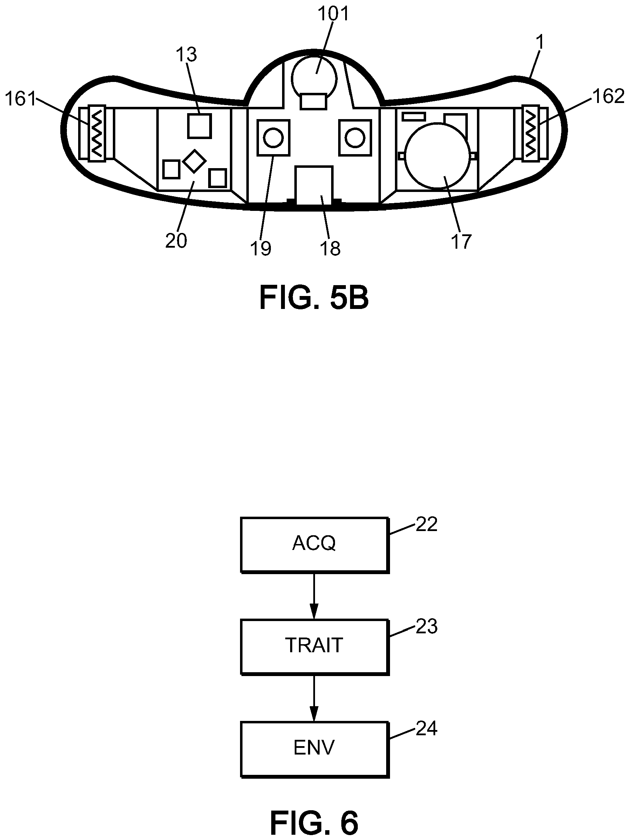

La **figure 5A** est un schéma représentant des composants présents dans le dispositif 1. Plusieurs capteurs 101, 102, 103, ..., 10N sont compris dans le boîtier du dispositif. Dans un mode de réalisation décrit ci-après en référence à la figure 5B, deux capteurs sont présents. Ces capteurs sont en charge de l'acquisition d'au moins une mesure physiologique du corps vivant.

Ces capteurs sont constitués d'une interface thermiquement conductrice et biocompatible (métal type inox 316L ou élastomère chargé), d'une thermistance (ou thermocouple) dont les propriétés électriques varient selon la température, et d'une couche d'isolation thermique afin d'isoler le capteur de l'environnement extérieur.

Le boîtier comprend en outre un multiplexeur 11 des données de mesure acquises par les capteurs. Ces données reçoivent ensuite un traitement analogique par un circuit analogique de traitement 12. Les données de mesures sont également converties dans un format numérique par le circuit 12. Un microcontrôleur 13 traite alors ces données, par exemple pour pondérer une mesure comme cela est décrit ci-après en référence à la figure 5B, ou pour stocker temporairement ces données afin que le dispositif envoie les données par grappes, ou encore pour analyser les données et détecter d'éventuelles erreurs.

Dans un circuit de modulation 14, les données sont alors mises en forme, démodulées et amplifiées, de façon connue en soi, pour être converties en signaux présentant un format adapté à une transmission radiofréquence. Les signaux sont alors démultiplexés par un démultiplexeur 15 afin d'être transmis par des antennes 161, 162, ..., 16N.

La **figure 5B** illustre l'intérieur du dispositif 1, dans un mode de réalisation. Deux antennes, 161 et 162, sont comprises à deux extrémités du dispositif. Comme mentionné ci-avant, lorsque le dispositif est en position d'utilisation, ces extrémités sont localisées sur et/ou dans un voisinage de portions de peau spécifiques.

Le boîtier comporte en outre une batterie 17, un interrupteur 19 et une prise multifonction 18. La prise multifonction 18, typiquement de type USB pour « universal serial bus », pour bus série universel en français, combine des fonctions de branchement pour recharger la batterie 17 et pour une connexion filaire avec un dispositif informatique externe. Typiquement, le dispositif informatique externe est une clé USB ou un ordinateur et la connexion avec ce dispositif externe est utilisée pour mettre à jour un programme informatique d'exploitation du microcontrôleur 13 et/ou d'un circuit imprimé 20.

Le circuit imprimé 20 comprend différents composants, dont le multiplexeur 11, le circuit analogique de traitement 12, le circuit de modulation 14 et le démultiplexeur 15. Certaines des fonctions assurées par le multiplexeur 11, le circuit analogique de traitement 12, le circuit de modulation 14 et le démultiplexeur 15 peuvent également être assurées par le microcontrôleur 13. Le circuit imprimé est flexible ou semi-flexible.

Dans un mode de réalisation, le circuit imprimé semi-flexible comprend deux couches de cuivre gravées de part et d'autre d'une couche de kapton, l'ensemble mesurant environ 200 µm d'épaisseur. Les composants (le microcontrôleur 13 typiquement) sont brasés d'un coté de ce circuit. Le circuit comprend en outre des renforts d'époxy de 500 µm d'épaisseur raccordés à la bande du coté opposé aux composants. Dans un autre mode de réalisation (technologie appelée « flex-rigid »), le circuit imprimé comprend une plaque d'époxy sur laquelle certaines parties ont été gravées plus profondément (ces parties font environ 200 µm d'épaisseur), ces parties plus fines conférant au circuit des propriétés de flexibilité.

Le microcontrôleur 13 et/ou le circuit imprimé 20 comprennent des circuits logiques programmables, tel qu'un FPGA, pour « field-programmable gâte array », réseau de portes programmables en français. Les programmes informatiques utilisés par de tels circuits logiques programmables sont chargés au moyen de la prise multifonction 18.

Le microcontrôleur 13 comprend au moins un processeur. Le microcontrôleur 13 peut également comprendre de la mémoire vive, de la mémoire de masse, ou encore le multiplexeur 11, le circuit analogique de traitement 12, le circuit de modulation 14 et le démultiplexeur 14.

Le dispositif 1 comprend en outre un premier capteur 101 de température dont au moins une portion est apte à être en contact avec la peau du corps humain lorsque le dispositif est dans la position d'utilisation, pour mesurer une température cutanée. Le dispositif 1 peut également comporter un deuxième capteur de température, non représenté sur la figure 5, dont au moins une portion est apte à être située à l'extérieur du boîtier lorsque le dispositif est dans la position d'utilisation, pour mesurer une température extérieure au boîtier. La température extérieure est utilisée pour pondérer la température cutanée. Les calculs de pondération sont effectués par le microcontrôleur 13.

Néanmoins, les deux mesures, effectuées par les deux capteurs, ont des constantes de temps différentes : la température extérieure au boîtier peut varier très rapidement, alors que la température cutanée va mettre plus de temps à réagir. La mesure pondérée peut tenir compte de cette différence de temps pour être réellement précise.

La **figure 6** illustre un procédé de transmission de mesures physiologiques acquises par le capteur 101 vers le terminal utilisateur 2. Lorsque le dispositif est dans la position d'utilisation, le procédé comporte une étape 22 d'acquisition d'au moins une mesure physiologique, une étape 23 de génération d'un signal radio fréquence comprenant la mesure et une étape 24 d'émission du signal par l'antenne selon le sens de rayonnement défini ci-avant.

La présente invention ne se limite pas aux formes de réalisation décrites ci-avant à titre d'exemples ; l'invention est définie par les revendications 1-11.

Ainsi, on a décrit ci-avant un mode de réalisation dans lequel la transmission des mesures était faite depuis un dispositif vers un terminal utilisateur. La transmission de ces mesures à plusieurs terminaux, simultanément ou non, est également envisageable. En outre, un terminal utilisateur peut recevoir, simultanément ou non, des mesures depuis plusieurs dispositifs d'acquisition.

De plus, on a décrit un mode de réalisation dans lequel la mesure physiologique est une mesure de température. D'autres mesures sont également envisageables, telles qu'une mesure de fréquence cardiaque, une mesure d'un taux de sucre dans le sang, une mesure d'un taux de sel dans la sueur, etc.

## Revendications

1. Dispositif d'acquisition (1) d'au moins une mesure physiologique d'un corps vivant (3), le corps vivant étant un corps humain, comportant au moins deux antennes directives pour une connexion sans fil avec un terminal utilisateur (2), le dispositif étant destiné à être en contact avec le corps vivant suivant une surface de contact dans une position d'utilisation, dans lequel chacune des antennes du dispositif a une directivité définie par un vecteur de rayonnement (*̅v̅_̅{̅R̅}̅*̅) respectif comportant une direction de rayonnement et un sens de rayonnement,
dans lequel, chacune des antennes est configurée pour rayonner selon la direction de rayonnement de son vecteur de rayonnement, le sens de rayonnement étant opposé à une portion du corps vivant sur laquelle elle est posée lorsque le dispositif est dans la position d'utilisation,
dans lequel, la surface de contact est apte à être située sensiblement sous une aisselle du corps humain et à s'étendre sur une portion de peau du tronc du corps humain lorsque le dispositif est dans la position d'utilisation,
dans lequel une première antenne directive est située au niveau d'une première extrémité du dispositif et une deuxième antenne directive est située au niveau d'une deuxième extrémité du dispositif, opposée à la première extrémité du dispositif,
dans lequel les au moins deux antennes ont des directivités respectives distinctes, chaque antenne définissant un champ de rayonnement, et
dans lequel le dispositif est apte à déterminer que le terminal utilisateur se situe dans le champ de rayonnement d'une antenne parmi les première et deuxième antennes, et dans lequel seule ladite antenne est activée.

2. Dispositif selon la revendication 1, dans lequel la portion du corps vivant est un organe vital.

3. Dispositif selon l'une des revendications 1 et 2,
dans lequel la première antenne est située au niveau d'une extrémité du dispositif apte à s'étendre sur une portion de peau située au-dessus d'un muscle pectoral lorsque le dispositif est dans la position d'utilisation et la deuxième antenne est située au niveau d'une autre extrémité du dispositif lorsque le dispositif est dans la position d'utilisation apte à s'étendre sur au moins un élément parmi :
• une portion de peau située au-dessus d'un muscle deltoïde ;
• une portion de peau située au-dessus d'un muscle térès majeur ;
• une portion de peau située au-dessus d'un muscle térès mineur ;
• une portion de peau située au-dessus d'un os omoplate.

4. Dispositif selon l'une des revendications 1 à 3, dans lequel, lorsque le dispositif est dans la position d'utilisation, la direction de rayonnement est définie par un axe sensiblement parallèle au plan médian (4) du corps.

5. Dispositif selon la revendication 4, dans lequel l'axe passe par l'aisselle.

6. Dispositif selon l'une des revendications précédentes, dans lequel le contact entre le dispositif et le corps vivant est apte à être assuré par une bande adhésive double face placée entre le dispositif et le corps vivant.

7. Dispositif selon l'une des revendications précédentes, comportant un boîtier étanche comportant au moins un capteur de la mesure physiologique et lesdites antennes.

8. Dispositif selon la revendication 7, et comportant :
• au moins un premier capteur de température dont au moins une portion est apte à être en contact avec la peau du corps humain lorsque le dispositif est dans la position d'utilisation, pour mesurer une température cutanée ;
• au moins un deuxième capteur de température dont au moins une portion est apte à être située à l'extérieur du boîtier lorsque le dispositif est dans la position d'utilisation, pour mesurer une température extérieure au boîtier ;
• un microcontrôleur pour pondérer la température cutanée en fonction de la température extérieure au boîtier.

9. Dispositif selon l'une des revendications 7 et 8, dans lequel le boîtier est réalisé dans une matière plastique flexible.

10. Procédé de transmission d'au moins une mesure physiologique d'un corps vivant (3), le corps vivant étant un corps humain, d'un dispositif d'acquisition vers un terminal utilisation, le dispositif comportant une antenne pour une connexion sans fil avec le terminal utilisateur, le dispositif étant destiné à être en contact avec le corps vivant suivant une surface de contact dans une position d'utilisation,
dans lequel le dispositif comporte au moins deux antennes directives,
dans lequel chacune des antennes a une directivité définie par un vecteur de rayonnement respectif comportant une direction de rayonnement et un sens de rayonnement,
dans lequel une première antenne directive est située au niveau d'une première extrémité du dispositif et une deuxième antenne directive est située au niveau d'une deuxième extrémité du dispositif, opposée à la première extrémité du dispositif,
dans lequel les au moins deux antennes ont des directivités respectives distinctes, chaque antenne définissant un champ de rayonnement, et
dans lequel, lorsque le dispositif est dans la position d'utilisation, le procédé comporte les étapes de :
- acquisition (22) d'au moins une mesure physiologique du corps vivant ;
- détermination que le terminal utilisateur se situe dans le champ de rayonnement d'une antenne parmi les première et deuxième antennes ;
- génération (23) d'un signal radiofréquence comprenant ladite mesure ;
- émission (24) dudit signal par ladite antenne selon un sens de rayonnement opposé à au moins une portion du corps vivant, ladite antenne étant la seule antenne activée parmi les première et deuxième antennes.

11. Programme informatique comportant des instructions pour la mise en œuvre du procédé selon la revendication 10, lorsque ces instructions sont exécutées par un processeur (13) d'un dispositif d'acquisition (1) d'au moins une mesure physiologique d'un corps vivant (3) selon l'une quelconque des revendications 1 à 9.

## Patentansprüche

1. Vorrichtung (1) zum Erfassen mindestens einer physiologischen Messgröße eines lebenden Körpers (3), wobei der lebende Körper ein menschlicher Körper ist, umfassend mindestens zwei Richtantennen zur drahtlosen Verbindung mit einem Benutzerterminal (2), wobei die Vorrichtung dazu bestimmt ist, in einer Benutzungsposition mit dem lebenden Körper entlang einer Kontaktfläche in Kontakt zu sein,
wobei jede der Antennen der Vorrichtung eine Richtungscharakteristik hat, die durch einen jeweiligen Strahlungsvektor (*̅v̅_̅{̅R̅}̅*̅) mit einer Strahlungsachse und einer Strahlungsrichtung definiert ist,
wobei jede der Antennen so konfiguriert ist, dass sie entlang der Strahlungsachse ihres Strahlungsvektors strahlt, wobei die Strahlungsrichtung einem Abschnitt des lebenden Körpers entgegengesetzt ist, auf dem sie platziert ist, wenn sich die Vorrichtung in der Benutzungsposition befindet,
wobei die Kontaktfläche geeignet ist, sich im Wesentlichen in einer Achselhöhle des menschlichen Körpers zu befinden und sich über einen Abschnitt der Haut des Rumpfes des menschlichen Körpers zu erstrecken, wenn sich die Vorrichtung in der Benutzungsposition befindet,
wobei eine erste Richtantenne im Bereich eines ersten Endes der Vorrichtung angeordnet ist und eine zweite Richtantenne im Bereich eines zweiten Endes der Vorrichtung, das dem ersten Ende der Vorrichtung gegenüber liegt, angeordnet ist,
wobei die mindestens zwei Antennen jeweils unterschiedliche Richtungscharakteristiken aufweisen, wobei jede Antenne ein Strahlungsfeld definiert, und
wobei die Vorrichtung geeignet ist, zu bestimmen, dass sich der Benutzerterminal innerhalb des Strahlungsfeldes einer der ersten und zweiten Antennen befindet, und wobei nur die besagte Antenne aktiviert ist.

2. Vorrichtung nach Anspruch 1, wobei der Teil des lebenden Körpers ein lebenswichtiges Organ ist.

3. Vorrichtung nach Anspruch 1 oder 2, wobei sich die erste Antenne im Bereich eines Endes der Vorrichtung befindet, der geeignet ist, sich über einen Abschnitt der Haut, die sich über einem Brustmuskel befindet, zu erstrecken, wenn sich die Vorrichtung in der Benutzungsposition befindet, und sich die zweite Antenne im Bereich eines anderen Endes der Vorrichtung befindet, wenn sich die Vorrichtung in der Benutzungsposition befindet, der geeignet ist, sich über mindestens eines von Folgendem zu erstrecken:
• einen Abschnitt der Haut, die sich über einem Deltamuskel befindet;
• einen Abschnitt der Haut, die sich über einem Musculus teres major befindet;
• einen Abschnitt der Haut, die sich über einem Musculus teres minor befindet;
• einen Abschnitt der Haut, die sich über einem Scapula-Knochen befindet.

4. Vorrichtung nach einem der Ansprüche 1 bis 3, wobei in der Benutzungsposition der Vorrichtung die Strahlungsachse durch eine Achse definiert ist, die im Wesentlichen parallel zur Medianebene (4) des Körpers verläuft.

5. Vorrichtung nach Anspruch 4, wobei die Achse durch die Achselhöhle verläuft.

6. Vorrichtung nach einem der vorhergehenden Ansprüche, wobei der Kontakt zwischen der Vorrichtung und dem lebenden Körper durch ein doppelseitiges Klebeband sichergestellt werden kann, das zwischen der Vorrichtung und dem lebenden Körper angebracht ist.

7. Vorrichtung nach einem der vorhergehenden Ansprüche, umfassend ein abgedichtetes Gehäuse mit mindestens einem Sensor für die physiologische Messgröße und mit den Antennen.

8. Vorrichtung nach Anspruch 7, umfassend:
• mindestens einen ersten Temperatursensor, von dem mindestens ein Abschnitt geeignet ist, mit der Haut des menschlichen Körpers in Kontakt zu sein, wenn die Vorrichtung in der Benutzungsposition ist, um eine Hauttemperatur zu messen;
• mindestens einen zweiten Temperatursensor, von dem mindestens ein Abschnitt geeignet ist, sich außerhalb des Gehäuses zu befinden, wenn die Vorrichtung in der Benutzungsposition ist, um eine Temperatur außerhalb des Gehäuses zu messen;
• einen Mikrocontroller, um die Hauttemperatur in Abhängigkeit von der Temperatur außerhalb des Gehäuses zu gewichten.

9. Vorrichtung nach einem der Ansprüche 7 und 8, wobei das Gehäuse aus einem flexiblen Kunststoffmaterial gefertigt ist.

10. Verfahren zur Übertragung mindestens einer physiologischen Messgröße eines lebenden Körpers (3), wobei der lebende Körper ein menschlicher Körper ist, von einer Erfassungsvorrichtung zu einem Benutzungsterminal, wobei die Vorrichtung eine Antenne zur drahtlosen Verbindung mit dem Benutzerterminal umfasst, wobei die Vorrichtung dazu bestimmt ist, mit dem lebenden Körper entlang einer Kontaktfläche in einer Benutzungsposition in Kontakt zu sein,
wobei die Vorrichtung mindestens zwei Richtantennen umfasst,
wobei jede der Antennen eine Richtungscharakteristik hat, die durch einen jeweiligen Strahlungsvektor mit einer Strahlungsachse und einer Strahlungsrichtung definiert ist,
wobei eine erste Richtantenne im Bereich eines ersten Endes der Vorrichtung angeordnet ist und eine zweite Richtantenne im Bereich eines zweiten Endes der Vorrichtung, das dem ersten Ende der Vorrichtung gegenüberliegt, angeordnet ist,
wobei die mindestens zwei Antennen jeweils unterschiedliche Richtungscharakteristiken aufweisen, wobei jede Antenne ein Strahlungsfeld definiert, und
wobei das Verfahren, wenn sich die Vorrichtung in der Benutzungsposition befindet, die folgenden Schritte umfasst:
- Erfassen (22) mindestens einer physiologischen Messgröße des lebenden Körpers;
- Bestimmen, dass sich der Benutzerterminal innerhalb des Strahlungsfeldes einer der ersten und zweiten Antennen befindet,
- Erzeugen (23) eines Hochfrequenzsignals, das die Messgröße umfasst;
- Ausgeben (24) des Signals durch die Antenne entlang einer Strahlungsrichtung, die mindestens einem Abschnitt des lebenden Körpers entgegengesetzt ist, wobei die besagte Antenne die einzige Antenne ist, die unter der ersten und der zweiten Antenne aktiviert ist.

11. Computerprogramm umfassend Anweisungen zur Durchführung des Verfahrens nach Anspruch 10, wenn diese Anweisungen von einem Prozessor (13) einer Vorrichtung (1) zum Erfassen mindestens einer physiologischen Messgröße eines lebenden Körpers (3) nach einem der Ansprüche 1 bis 9 ausgeführt werden.

## Claims

1. Device (1) for acquiring at least one physiological measurement of a living body (3), the living body being a human body, including at least two directional antennas for a wireless connection with a user terminal (2), the device being intended to be in contact with the living body along a contact surface in a position of use,
wherein each of the antennas of the device has a directivity that is defined by a respective radiation vector (*̅v̅_̅{̅R̅}̅*̅) including an orientation of radiation and a sense of radiation,
wherein each of the antennas is configured to radiate according to the orientation of radiation of its radiation vector, the sense of radiation being opposite a portion of the living body on which it is placed when the device is in the position of use,
wherein the contact surface is capable of being located substantially underneath an axilla of the human body and of extending over a portion of skin of the trunk of the human body when the device is in the position of use,
wherein a first directional antenna is located at a first end of the device and a second directional antenna is located at a second end of the device, opposite the first end of the device,
wherein the at least two antennas have separate respective directivities, each antenna defining a radiation field, and
wherein the device is capable of determining that the user terminal is located within the radiation field of one of the first and second antennas, and wherein only said antenna is activated.

2. Device according to claim 1, wherein the portion of the living body is a vital organ.

3. Device according to any one of claims 1 and 2, wherein the first antenna is located at one end of the device and is capable of extending over a portion of skin located above a pectoral muscle when the device is in the position of use and the second antenna is located at another end of the device when the device is in the position of use and is capable of extending over at least one from among:
- a portion of skin located above a deltoid muscle;
- a portion of skin located above a teres major muscle;
- a portion of skin located above a teres minor muscle;
- a portion of skin located above a scapula bone.

4. Device according to any one of claims 1 to 3, wherein, when the device is in the position of use, the orientation of radiation is defined by an axis substantially parallel to the median plane (4) of the body.

5. Device according to claim 4, wherein the axis passes through the axilla.

6. Device according to any one of the preceding claims, wherein the contact between the device and the living body is capable of being procured by a double-sided adhesive tape placed between the device and the living body.

7. Device according to any one of the preceding claims, including an impervious housing including at least one physiological measurement sensor and said antennas.

8. Device according to claim 7, and including:
- at least one first temperature sensor, at least a portion whereof is capable of being in contact with the skin of the human body when the device is in the position of use, to measure a skin temperature;
- at least one second temperature sensor, at least a portion whereof is suitable for being located outside the housing when the device is in the position of use, to measure a temperature outside the housing;
- a microcontroller to weight the skin temperature as a function of the temperature outside the housing.

9. Device according to any one of claims 7 and 8, wherein the housing is made of a flexible plastics material.

10. Method for transmitting at least one physiological measurement of a living body (3), the living body being a human body, from an acquisition device to a user terminal, the device including an antenna for a wireless connection with the user terminal, the device being intended to be in contact with the living body along a contact surface in a position of use,
wherein the device includes at least two directional antennas,
wherein each of the antennas has a directivity that is defined by a respective radiation vector including an orientation of radiation and a sense of radiation,
wherein a first directional antenna is located at a first end of the device and a second directional antenna is located at a second end of the device, opposite the first end of the device,
wherein the at least two antennas have separate respective directivities, each antenna defining a radiation field, and
wherein, when the device is in the position of use, the method includes the steps of:
- acquiring (22) at least one physiological measurement of the living body;
- determining that the user terminal is located within the radiation field of one of the first and second antennas;
- generating (23) a radio frequency signal comprising said measurement;
- transmitting (24) said signal by said antenna according to a sense of radiation opposite to at least a portion of the living body, said antenna being the only antenna activated from among the first and second antennas.

11. Computer program including instructions for implementing the method according to claim 10, when these instructions are executed by a processor (13) of a device (1) for acquiring at least one physiological measurement of a living body (3) according to any one of claims 1 to 9.
